# EUROPEAN PATENT SPECIFICATION

(11) **EP 4 524 220 B1**
(45) Date of publication and mention of the grant of the patent: **29.07.2026**
(21) Application number: 24201207.8
(22) Date of filing: 18.09.2024
(51) Int. Cl.: C10L 3/08, C10L 3/10

(54) **CARBON DIOXIDE (CO2) MANAGEMENT IN CARBON-CARBON (C/C) PREFORM PRODUCTION PROCESS**
KOHLENDIOXID (CO2)-MANAGEMENT IN EINEM KOHLENSTOFF-KOHLENSTOFF (C/C)-VORFORMHERSTELLUNGSVERFAHREN
GESTION DU DIOXYDE DE CARBONE (CO2) DANS UN PROCÉDÉ DE PRODUCTION DE PRÉFORME DE CARBONE-CARBONE (C/C)

(30) Priority: 18.09.2023 US 202318469139
(43) Date of publication of application: 19.03.2025
(73) Proprietor: Goodrich Corporation, Charlotte, NC 28217-4578 (US)
(72) Inventor: CORDATOS, Haralambos, Colchester, CT (US); DARDAS, Zissis A., Worcester, MA (US); EMERSON, Sean C., Broad Brook, CT (US); SHE, Ying, Rocky Hill, CT (US); SUTTON, Leah Patrice, Pueblo, CO (US); YOUNG, Michael N., Colorado Springs, CO (US); HALL, Daniel, Pueblo, CO (US)
(74) Representative: Dehns

(56) References cited:
- EP-B1- 3 530 640
- WO-A1-2021/246318
- JP-A- 2005 221 150

## Description

### FIELD

The present disclosure relates generally to carbon dioxide (CO₂) emission recovery, and more particularly, CO₂ emission recovery in carbon/carbon (C/C) preform production process.

### BACKGROUND

Composite bodies are utilized in various industries, including the aerospace industry. The composite bodies start with a preform that is formed using layers of textile material. Aircraft C/C brake manufacturing is one point source of carbon dioxide (CO₂) emissions. Other point sources of CO₂ emissions may include thermal protections systems and shaped composite bodies, among others. In such manufacturing, sustainability goals may be to reduce CO₂ emissions by a predetermined percentage by a future date. However, capturing CO₂ emissions is not only expensive but CO₂ emissions can also be problematic to store. This is especially true for CO₂ emission sources that do not generate enough CO₂ emissions to justify use of a pipeline for underground sequestration. EP 3 530 640 B1 discloses a methane production device and a methane production method. WO 2021/246318 A1 relates to a method for producing methane from CO₂ in cement production exhaust gas and a methanation apparatus. JP 2005-221150 relates to waste gas treatment device for carbon fiber manufacturing device by vapor-phase method.

### SUMMARY

The invention is defined in the appended claims.

A system for carbon dioxide (CO₂) emission recovery is provided and defined in claim 1. The system includes a methanization reactor. The methanization reactor is configured to receive CO₂ separated from one or more off gases of a carbon/carbon (C/C) preform production process; convert the CO₂ and supplied hydrogen (H₂) via methanization to produce methane (CH₄); and supply the produced CH₄ to at least one of the carbon/carbon (C/C) preform production process or another system for heat generation. The system includes an H₂ separator. The supplied H₂ is separated by the H₂ separator from the one or more off gases produced from the C/C preform production process.

In various embodiments, the system includes a green H₂ supply device. In various embodiments, the supplied H₂ is green H₂ supplied via the green H₂ supply device. In various embodiments, the green H₂ supply device is at least one of a solar generator or wind generator. In various embodiments, the system includes an H₂ separator and a green H₂ supply device. In various embodiments, a portion of the supplied H₂ is separated by the H₂ separator from the one or more off gases produced from the C/C preform production process and is supplemented by green H₂ supplied via the green H₂ supply device. In various embodiments, the green H₂ supply device is a solar generator or wind generator.

In various embodiments, the system includes an H₂ separator and a burner/steam generator. In various embodiments, the supplied H₂ is separated by the H₂ separator from the one or more off gases produced from the C/C preform production process and remaining off gases from the separation of the supplied H₂ are fed to the burner/steam generator. In various embodiments, the burner/steam generator generates at least the CO₂ that is used by the methanization reactor. In various embodiments, the burner/steam generator further generates steam. In various embodiments, the steam is supplied to the C/C preform production process. In various embodiments, the system includes a separator. In various embodiments, the burner/steam generator generates byproducts and the separator separates the CO₂ from the byproducts and provides the CO₂ to the methanization reactor. In various embodiments, the separator further separates nitrogen (N₂) from the byproducts and releases the N₂ into an atmosphere. In various embodiments, separator further separates water (H₂O) from the byproducts and feeds the H₂O into the burner/steam generator.

In various embodiments, the system includes a burner/steam generator and a condenser. In various embodiments, converting the CO₂ and the supplied hydrogen (H₂) via methanization produces the CH₄ and steam. In various embodiments, the condenser condenses the steam to water (H₂O) which is supplied to the burner/steam generator.

A method for carbon dioxide (CO₂) emission recovery is provided as defined by claim 9. The method includes receiving, by a methanization reactor, CO₂ separated from one or more off gases of a carbon/carbon (C/C) preform production process; converting, by the methanization reactor, the CO₂ and supplied hydrogen (H₂) via methanization to produce methane (CH₄); and supplying, by the methanization reactor, the produced CH₄ to at least one of the carbon/carbon (C/C) preform production process or another system for heat generation.

In various embodiments, the supplied H₂ is separated, by an H₂ separator, from the one or more off gases produced from the C/C preform production process prior to the methanization reactor converting the CO₂ and the supplied H₂ via methanization to produce the CH₄. In various embodiments, the supplied H₂ is green H₂ supplied via a green H₂ supply device. In various embodiments, the green H₂ supply device is at least one of a solar generator or wind generator. In various embodiments, a portion of the supplied H₂ is provided by separating, by an H₂ separator, the supplied H₂ from the one or more off gases produced from the C/C preform production process prior to the methanization reactor converting the CO₂ and the supplied H₂ via methanization to produce the CH₄. In various embodiments, the portion of the supplied H₂ is supplemented by green H₂ supplied via a green H₂ supply device. In various embodiments, the green H₂ supply device is a solar generator or wind generator.

In various embodiments, the supplied H₂ is separated, by a H₂ separator, from the one or more off gases produced from the C/C preform production process and remaining off gases from the separation of the supplied H₂ are fed to a burner/steam generator. In various embodiments, at least the CO₂ is generated by the burner/steam generator and used by the methanization reactor. In various embodiments, steam is generated by the burner/steam generator. In various embodiments, the steam is supplied to the C/C preform production process. In various embodiments, the burner/steam generator generates byproducts. In various embodiments, a separator separates the CO₂ from the byproducts and provides the CO₂ to the methanization reactor. In various embodiments, the separator further separates nitrogen (N₂) from the byproducts and releases the N₂ into an atmosphere. In various embodiments, the separator further separates water (H₂O) from the byproducts and feeds the H₂O into the burner/steam generator. In various embodiments, converting the CO₂ and the supplied hydrogen (H₂) via methanization produces the CH₄ and steam. In various embodiments, a condenser condenses the steam to water (H₂O) which is supplied to a burner/steam generator.

The forgoing features and elements may be combined in various combinations without exclusivity, unless expressly indicated herein otherwise. These features and elements as well as the operation of the disclosed embodiments will become more apparent in light of the following description and accompanying drawings.

### BRIEF DESCRIPTION OF THE DRAWINGS

FIG. 1A illustrates an exemplary aircraft having a brake system, in accordance with various embodiments.
FIG. 1B illustrates a cross-sectional view of a brake assembly, in accordance with various embodiments.
FIG. 2 illustrates a fibrous preform, in accordance with various embodiments.
FIG. 3 illustrates a fibrous preform in a carbonization furnace, in accordance with various embodiments.
FIG. 4 illustrates a system for CO₂ emission recovery by converting recovered CO₂ into other products, in accordance with various embodiments.
FIG. 5 illustrates a system for CO₂ emission recovery by converting recovered CO₂ into other products, in accordance with various embodiments.
FIG. 6 illustrates a system for CO₂ emission recovery by converting recovered CO₂ into other products, in accordance with various embodiments.
FIG. 7 illustrates a method for CO₂ emission recovery by converting recovered CO₂ into other products, in accordance with various embodiments.

### DETAILED DESCRIPTION

The following detailed description of various embodiments herein makes reference to the accompanying drawings, which show various embodiments by way of illustration. While these various embodiments are described in sufficient detail to enable those skilled in the art to practice the disclosure, it should be understood that other embodiments may be realized and that changes may be made without departing from the scope of the disclosure. Thus, the detailed description herein is presented for purposes of illustration only and not of limitation. Furthermore, any reference to singular includes plural embodiments, and any reference to more than one component or step may include a singular embodiment or step. Also, any reference to attached, fixed, connected, or the like may include permanent, removable, temporary, partial, full or any other possible attachment option. Additionally, any reference to without contact (or similar phrases) may also include reduced contact or minimal contact. It should also be understood that unless specifically stated otherwise, references to "a," "an," or "the" may include one or more than one and that reference to an item in the singular may also include the item in the plural. Further, all ranges may include upper and lower values and all ranges and ratio limits disclosed herein may be combined.

In some typical carbon/carbon (C/C) preform production process, such as aircraft C/C brake manufacturing, thermal protections systems, and shaped composite bodies, among others, carbon dioxide (CO₂) emissions account for a substantial percentage of a total CO₂ emissions for some corporations. In such corporations, sustainability goals may be to decarbonize operations, e.g., by reducing CO₂ emissions, by a predetermined percentage by a future date. While technology for capturing CO₂ emissions may be well-established and already in practice for applications ranging from enhanced oil recovery to carbonation of soft drinks, one of the costs associated with capturing CO₂ emissions is the logistics and additional energy input associated with CO₂ emission sequestration, which, for large point sources such as power plants, involves pressurization and transfer via pipeline to underground storage locations. Such CO₂ emission recovery is unlikely to become a viable solution for relatively small CO₂ emission point sources.

Disclosed herein are systems and methods for CO₂ emission recovery by converting the recovered CO₂ into other products. Accordingly, in various embodiments, in manufacturing processes where other gases such as hydrogen (H₂) are also generated as a byproduct, the H₂ may react catalytically with the recovered CO₂ to form methane (CH₄) via a methanization reaction, i.e., the main species in natural gas:

CO₂ + 4H₂ → CH₄ + 2H₂O

This methanization reaction may also be referred to as a Sabatier reaction. In manufacturing processes, such as preform forming manufacturing, the methanization reaction offers the most straightforward path for management of CO₂ emissions, given that the preform forming manufacturing process already typically utilizes methane (natural gas) for operation and given that the C/C brake manufacturing process generates excess H₂. While the excess H₂ generated by the C/C brake manufacturing process may not be enough to account for the entire CO₂ emissions, the H₂ may be supplemented with additional "green hydrogen" (i.e., produced sustainably), which is projected to become widely available. Additionally, the water (H₂O) produced from the methanization reactor may also be recycled back into a steam generator used for the C/C brake manufacturing process.

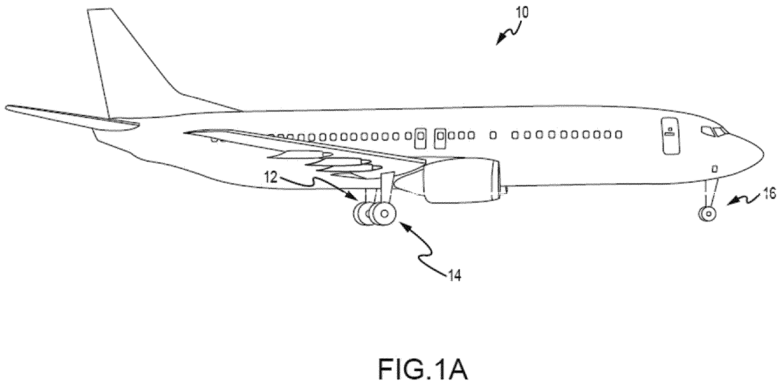

Referring now to FIG. 1A, in accordance with various embodiments, an aircraft 10 is illustrated. The aircraft 10 includes landing gear, which may include a left main landing gear 12, a right main landing gear 14, and a nose landing gear 16. The landing gear support the aircraft 10 when it is not flying, allowing the aircraft 10 to taxi, take off, and land without damage. While the disclosure refers to the three landing gear configurations just described, the disclosure nevertheless contemplates any number of landing gear configurations.

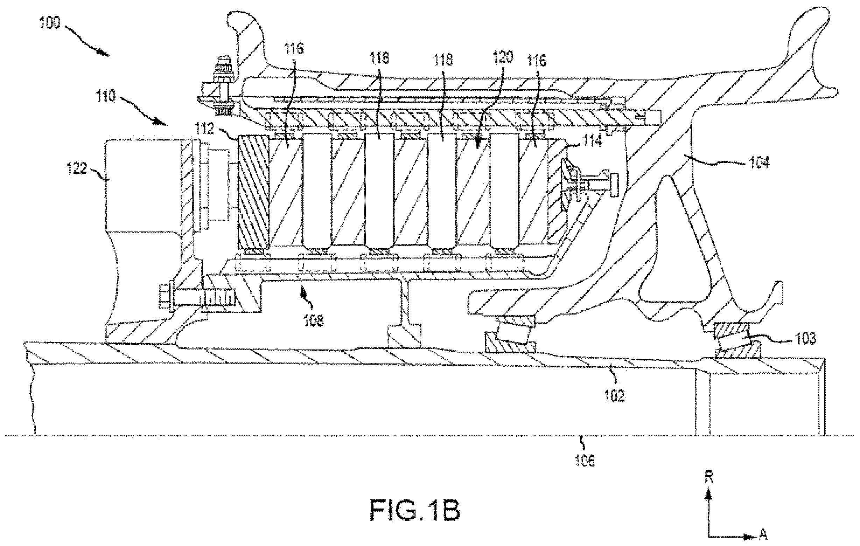

Referring now to FIG. 1B, in accordance with various embodiments, a schematically depicted a brake mechanism 100 is illustrated configured for use on a landing gear, such as, for example, each of the left main landing gear 12 and the right main landing gear 14 described above with reference to FIG. 1A. In various embodiments, the brake mechanism 100 is mounted on an axle 102 for use with a wheel 104 disposed on and configured to rotate about the axle 102 via one or more bearing assemblies 103. A central axis 106 extends through the axle 102 and defines a center of rotation of the wheel 104. As used herein, the term "radial" refers to directions perpendicular to a central axis 106 of fibrous preform, the term "axial" refers to direction parallel to central axis 106, and the term "circumferential" reference to directions about central axis 106. A torque plate barrel 108 (sometimes referred to as a torque tube or a torque plate) is aligned concentrically with the central axis 106, and the wheel 104 is rotatable relative to the torque plate barrel 108.

The brake mechanism 100 includes a piston assembly 110, a pressure plate 112 disposed adjacent the piston assembly 110, an end plate 114 positioned a distal location from the piston assembly 110, and a plurality of rotor disks 116 interleaved with a plurality of stator disks 118 positioned intermediate the pressure plate 112 and the end plate 114. The pressure plate 112, the plurality of rotor disks 116, the plurality of stator disks 118, and the end plate 114 together form a brake heat sink or brake stack 120. The pressure plate 112, the end plate 114, and the plurality of stator disks 118 are mounted to the torque plate barrel 108 and remain rotationally stationary relative to the axle 102. The plurality of rotor disks 116 is mounted to the wheel 104 and rotate with respect to each of the pressure plate 112, the end plate 114, and the plurality of stator disks 118.

An actuating mechanism for the brake mechanism 100 includes a plurality of piston assemblies, including the piston assembly 110, circumferentially spaced around a piston housing 122 (only one piston assembly is illustrated in FIG. 1B). Upon actuation, the plurality of piston assemblies affects a braking action by urging the pressure plate 112 and the plurality of stator disks 118 into frictional engagement with the plurality of rotor disks 116 and against the end plate 114. Through compression of the plurality of rotor disks 116 and the plurality of stator disks 118 between the pressure plate 112 and the end plate 114, the resulting frictional contact slows or stops or otherwise prevents rotation of the wheel 104. In various embodiments, the brake disks of brake mechanism 100 (e.g., rotor disks 116 and the stator disks 118) are fabricated from various composite materials, such as, for example, carbon/carbon (C/C) composite or ceramic matrix composite (CMCs), that enable the brake disks to withstand and dissipate the heat generated during and following a braking action.

In accordance with various embodiments, rotor disks 116 and/or stator disks 118 are each comprised of a carbon-carbon (C/C) material having a high specific heat particulate interspersed throughout the rotor disks 116 and/or stator disks 118, where high specific heat particulate includes any particulate or powder (typically ceramic) that raises the specific heat of the disk above that of C/C composite alone. For example, in various embodiments, the rotor disks 116 and/or stator disks 118 may comprise a C/C composite that includes a percentage of boron, a boron component, or other material having a high specific heat (i.e., a specific heat greater than the specific heat of the C/C composite alone). In various embodiments, rotor disks 116 and/or stator disks 118 may comprise a C/C composite with a percentage of boron carbide (B₄C) disposed substantially throughout the disk.

In various embodiments, the process of interspersing the boron carbide (or other high specific heat component) into the C/C composite is performed by a slurry infiltration process. As described in further detail below, the slurry may infiltrate a fiber preform employed to form the C/C composite in the radial, or in-plane, direction. The slurry may infiltrate via through thickness infiltration of the carbonize preforms. In various embodiments infiltrating in the radial, as opposed to the axial, direction may allow greater volumes and/or larger size particles to be infiltrated. Increasing the volume and/or size of the particles may facilitate the densification process by increasing the surface area available for the matrix material to bond to and by decreasing the open, or empty, volume within the preform. In-plane infiltration may also allow thicker fibrous preforms (e.g., fibrous preforms including a greater number of fiber layers) to be used in the manufacture rotor disks 116 and/or stator disks 118, as the compared to axial infiltration.

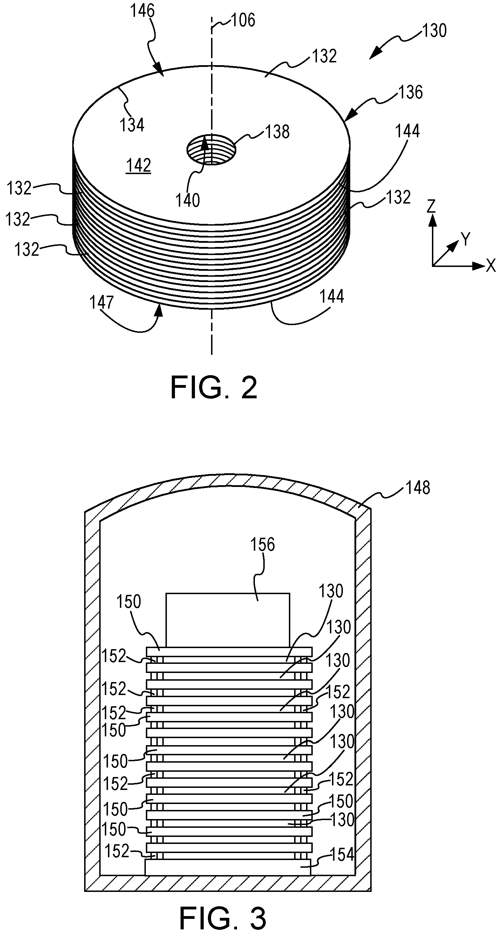

Referring now to FIG. 2, in accordance with various embodiments, a fibrous preform 130 is illustrated. Fibrous preform 130 may be employed to form a rotor disk 116 or a stator disk 118, as described above. Fibrous preform 130 may comprise a porous structure comprised of a plurality of stacked textile layers 132. Each textile layer 132 having a thickness in a first dimension (i.e., the Z-direction) that may be substantially less than a thickness of the layer 132 in the other two dimensions (i.e., the X-direction and the Y-direction). As used herein, the "in-plane" direction refers to directions parallel to the thicker two dimensions (i.e., parallel to the X and Y directions and perpendicular to the Z-direction).

A porous structure may comprise any structure derived from a fibrous material such as carbon fibers or the like. In various embodiments, the carbon fibers may be derived from polyacrylonitrile (PAN), rayon (synthetic fiber derived from cellulose), oxidized polyacrylonitrile fiber (OPF), pitch, or the like. The starting fiber may be pre-oxidized PAN or fully carbonized commercial carbon fiber. Fibrous preform 130 may be prepared by needling the textile layers 132 of fibrous preform 130. Needling the textile layers 132 of fibrous preform 130 tends to push fibers from one layer 132 to the next layer 132, thereby forming z-fibers that extend axially across the layers 132. Needling pulls fibers from the in-plane direction and forces the fibers into the z-fiber direction. After needling, fibrous preform 130 may comprise fibers extending in three different directions: the radial direction, the circumferential direction, and the axial direction (or the X, Y, and Z directions).

Fibrous preform 130 may be fabricated using a net shape preforming technology or may be cut from a needled board. Fibrous preform 130 may be a lay-up of woven, braided or knitted textile layers 132. The fibrous material may be in the form of chopped carbon fibers molded to form layers 132. Prior to the densification process, the fibrous material may be formed into a preform having any desired shape or form. For example, the fibrous preform may be in the form of a disk having any shape such as, for example, a polygon, a cylinder, a triangle, annular, square, rectangle, pentagon, hexagon, octagon, or the like. In various embodiments, layers 132 and fibrous preform 130 may have a generally annular shape. In accordance with various embodiments, the outer circumferential (or outer perimeter) surfaces 134 of layers 132 may form an outer diameter (OD) 136 of fibrous preform 130, and the inner circumferential (or inner perimeter) surfaces 138 of layers 132 may form an inner diameter (ID) 140 of fibrous preform 130. Each layer 132 includes a first axial face 142 and a second axial face 144 opposite the first axial face 142. First and second axial faces 142, 144 extend from outer circumferential surface 134 to inner circumferential surface 138. Layers 132 are stacked such that the first axial face 142 of one layer 132 is oriented toward the second axial face 144 of the adjacent layer 132. First axial face 142 of the uppermost layer 132 forms the upper axial end 146 of fibrous preform 130 and the second axial face 144 of the bottommost layer 132 forms the lower axial end 147 of fibrous preform 130 (i.e., the two layers 132 that are farther apart from one another in the axial direction form the axial ends 146, 147 of the fibrous preform).

As shown in FIG. 3, in accordance with various embodiments, fibrous preforms 130 being placed in a furnace 148 for carbonization is illustrated. The carbonization process may be employed to convert the fibers of the fibrous preforms 130 into pure carbon fibers, as used herein only "pure carbon fibers" means carbon fibers comprised of at least 99% carbon. The carbonization process is distinguished from the densification process described below in that the densification process involves infiltrating the pores of the fibrous preform 130 and depositing a matrix (e.g., carbon, phenolic resin, or any other desired matrix material) within and around the carbon fibers of the fibrous preform, and the carbonization process refers to the process of converting the fibers of the fibrous preform 130 into pure carbon fibers.

In various embodiments, a plurality of fibrous preforms 130 may be placed on top of one another with separator plates 150 and spacing stops 152 disposed between adjacent fibrous preforms 130. For example, the bottommost fibrous preform 130 may be placed on a base plate 154 at the bottom of carbonization furnace 148. A separator plate 150 may be placed on top of the bottommost fibrous preform 130. Another fibrous preform 130 may then be placed on the separator plate 150, and another separator plate 150 may be placed on that fibrous preform 130. A stack of fibrous preforms 130 and separator plates 150 may be constructed in this manner, with each fibrous preform 130 being separated from superjacent and subjacent fibrous preforms 130 by separator plates 150. Stops 152 may be placed between each of the separator plates 150. The stops 152 may comprise a height that is less than the thickness of the fibrous preform 130 prior to carbonization. Stops 152 may define a target thickness of the fibrous preform 130 after carbonization. In that regard, after the stack of fibrous preforms 130 is constructed, and before the carbonization process has started, gaps may exist between the stops 152 and adjacent separator plates 150. During carbonization, a compressive load may be applied to the fibrous preforms 130, thereby compressing the fibrous preforms 130 until stops 152 contact adjacent separator plates 150.

In various embodiments, compressive pressure may be applied to fibrous preforms 130 during the carbonization. The compressive pressure may be applied by placing a weight 156 over fibrous preforms 130, or by applying a compressive load to the fibrous preforms 130 by other suitable means. The compressive pressure may be applied along the direction of the z-fibers. It will be appreciated by those skilled in the art that the mass of weight 156 and/or the compressive force generated by weight 156 may vary depending on the size of fibrous preforms 130, the pre-carbonization fiber volume of fibrous preforms 130, the desired post-carbonization fiber volume of fibrous preforms 130, and/or the number fibrous preforms 130 being compressed. As used herein, "fiber volume" refers the percentage of the total volume of the fibrous preform that is formed by the fibers of the fibrous preform. For example, a fiber volume of 18% means the fibers of the fibrous preform form 18% of the total volume of fibrous preform. In various embodiments, after carbonization, fibrous preform 130 includes a fiber volume of between 10% and 50%. In various embodiments, after carbonization, fibrous preform 130 includes a fiber volume of between 15% and 25%. In various embodiments, fibrous preforms 130 having a low fiber volume may be desirable for the infiltration methods disclosed herein. In various embodiments, after carbonization, fibrous preform 130 may comprise a fiber volume of less than 25%. For example, in various embodiments, after carbonization, fibrous preform 130 may comprise a fiber volume of 21% or, in various embodiments, fibrous preform 130 may comprise a fiber volume of 18%.

After carbonization, fibrous preform 130, may be densified using, for example, CVI. In various embodiments, prior to densification, fibrous preform 130 is infiltrated with a slurry including a high specific heat particulate. For example, in various embodiments, fibrous preform 130 is infiltrated with a ceramic slurry (i.e., a slurry comprised of a liquid carrier and ceramic particulates). In various embodiments, the slurry infiltration process includes preparation of a slurry including a ceramic particulate (e.g., an aqueous B₄C-based slurry) and immersing the carbonized fibrous preform 130 into the slurry for a period of time sufficient for the particulate (e.g., the B₄C) to infiltrate the fibrous preform 130.

The slurry includes sacrificial fibers. For instance, the sacrificial fibers / particulates are mixed into the slurry. The sacrificial fibers may be a polyethylene or polyester fiber such that, in response to heating to carbon CVI temperatures (e.g., 100°F and 205°F (38°C and 96°C)) will burn. In various embodiments, the preform is heated up to 1000°C in order to cause the sacrificial fibers for burn away, or decompose. When the sacrificial fibers are burned away, or decompose, channels are formed, thus improving carbon CVI infiltration. For instance, the channels provide a pathway for the CVI to get to in. However, polyester or polyethylene fibers may be too large to incorporate directly into the B₄C slurry. Accordingly, the sacrificial fibers may be injected into the fibrous preform 130. For instance, the sacrificial fibers may be injected using a syringe or other means, prior to completing B₄C slurry infiltration. By injecting using a syringe, the sacrificial fibers may be distributed in the matrix of the boron carbide.

Slurry may include high specific heat particulate and a liquid carrier (such as, for example, water and/or alcohol). In various embodiments, slurry may further include a high specific heat particulate, a binder (e.g., a polymeric adhesive or polyvinyl acetate), and a liquid carrier (e.g., water). It will be appreciated by those skilled in the art that liquid carriers other than water may be used and that the type and/or volume of liquid carrier and/or of binder may be selected based on the composition of the high specific heat particulate. In various embodiments, slurry may be a B₄C-based slurry and may be prepared by mixing B₄C powder in water with appropriate additives, such as wetting agents and dispersants. The B₄C powder may comprise particulates having an average particle sizes from sub-micron up to about 30 microns. As used in the previous context only, "about" means + 5 microns.

The slurry further includes the sacrificial fibers. The sacrificial fibers are added to the solution of the slurry. In various embodiments, the sacrificial fibers make up 5% of the slurry solution. In various embodiments, the sacrificial fibers make up 10 % of the slurry solution. For instance, the sacrificial fibers make up .5% - 10% of the slurry. The sacrificial fibers may be a micro polyester particle. The slurry solution may be loaded into the syringe. A user may then use the syringe to inject the slurry, including the sacrificial fibers, into the fibrous preform 130. The user may inject the fibrous preform 130 in a plurality of locations. For instance, the user may inject/poke the fibrous preform 130 at ten locations. In various embodiments, a bank of syringes may be provided to operate as an assembly-line system to prepare multiple fibrous preforms may be injected simultaneously with the sacrificial fibers.

Infiltration using a syringe facilitates achieving a uniform distribution of the sacrificial fibers, which in turn improves carbon CVI infiltration once the fibers are burned around during the heat-up to carbon CVI process temperature. By raising the thermal capacity of the material, more energy may be absorbed per unit mass. Accordingly, the brakes may be manufactured smaller, for instance. Further, the friction and wear characteristics may be improved.

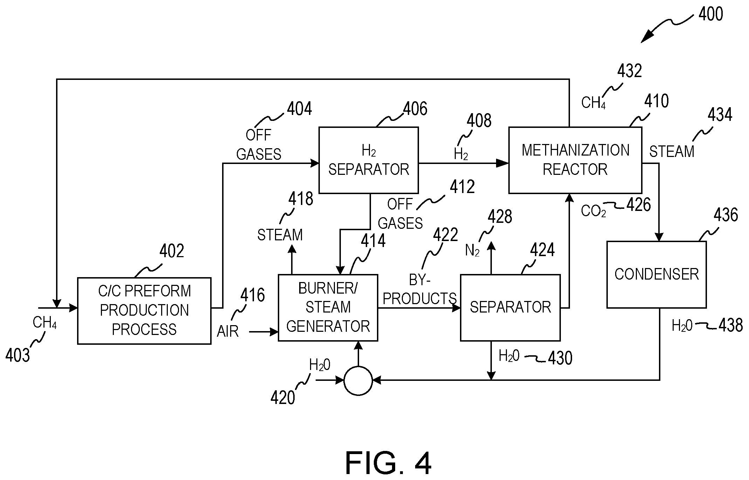

As described previously, the process illustrated and described with respect to FIG. 3 is the emission of off gases that include but are not limited to hydrogen (H₂) and carbon dioxide (CO₂). Referring now to FIG. 4, in accordance with various embodiments, a system 400 for CO₂ emission recovery by converting recovered CO₂ into other products is illustrated. In various embodiments, the C/C preform production process 402 utilizes natural gas such as methane (CH₄) 403 to perform various operations in the production of the aircraft brakes. However, in various embodiments, during the C/C preform production process 402, various off gases 404, i.e. hydrocarbons, are produced. Accordingly, in system 400, the C/C preform production process 402 feeds the various off gases 404, i.e., CO₂, H₂, and nitrogen (N₂), among others, into H₂ separator 406. The H₂ separator 406 separates H₂ 408 from the various off gases 404 using one or more of membrane separation, pressure swing absorption (PSA), captured by solid sorbents or solvents such as mono-ethanol amine (MEA), or cryogenic distillation, among others. In various embodiments, the H₂ separator 406 feeds the H₂ 408 into methanization reactor 410 and feeds the remaining off gases 412, i.e. heavier hydrocarbons, i.e., CO₂ and N₂, among others, into burner/steam generator 414.

In various embodiments, the burner/steam generator 414 burns the remaining off gases 412 utilizing air 416 to generate steam 418 from incoming water (H₂O) 420. In various embodiments, the steam 418 may be utilized to operate other components of the C/C preform production process 402. During the operations performed by the burner/steam generator 414, in addition to generating the steam 418, the burner/steam generator 414 generates byproducts 422, such as such as CO₂, H₂O, and N₂, among others, which the burner/steam generator 414 feeds into separator 424. Separator 424 may operate to separate the CO₂ 426 from the byproducts 422, which is fed into methanization reactor 410, to separate the N₂ 428 from the byproducts 422, which may be released into the atmosphere, and to separate the H₂O 430 from the byproducts 422, which may be fed into the burner/steam generator 414 along with H₂O 420. The separator 424 may include one or more of absorption, adsorption, cryogenic distillation, captured by solid sorbents or solvents such as mono-ethanol amine (MEA), or membrane separation, among others for the separation process.

In various embodiments, the methanization reactor 410 utilizes the incoming H₂ 408 and CO₂ 426 to perform a methanization process in order to produce methane (CH₄) 432, which is fed into the C/C preform production process 402 or another system to perform various operation in the production the aircraft brakes, such as heat generation. In various embodiments, in the production of CH₄ 432, the methanization reactor 410 also produces steam 434, which is fed into condenser 436. In various embodiments, the condenser 436 condenses the steam 434 to generate H₂O 438, which may be fed into the burner/steam generator 414 along with H₂O 420 and H₂O 430. Accordingly, system 400 provides for CO₂ emission recovery by converting the recovered CO₂ into other products that may be utilized by the C/C preform production process 402.

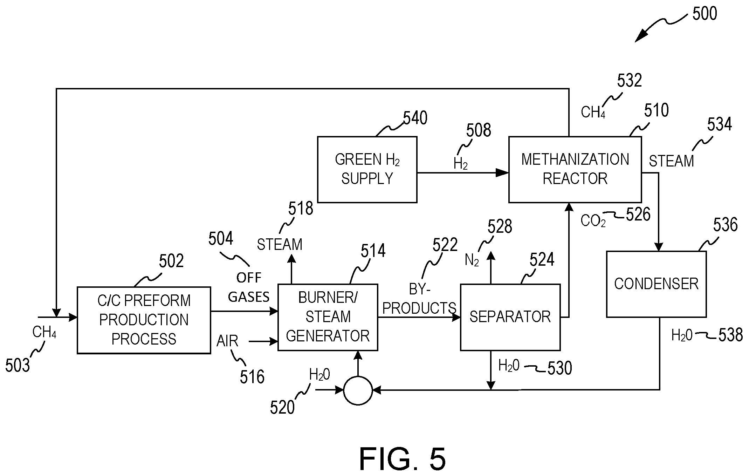

Referring now to FIG. 5, in accordance with various embodiments, a system 500 for CO₂ emission recovery by converting recovered CO₂ into other products is illustrated. In various embodiments, the C/C preform production process 502 utilizes natural gases such as methane (CH₄) 503 to perform various operation in the production the aircraft brakes. However, in various embodiments, during the C/C preform production process 502, various off gases 504, i.e. hydrocarbons, are produced. Accordingly, in system 500, the C/C preform production process 502 feeds the various off gases 504, i.e., CO₂, H₂, and nitrogen (N₂), among others, into burner/steam generator 514. In various embodiments, the burner/steam generator 514 burns the various off gases 504 utilizing air 516 to generate steam 518 from incoming water (H₂O) 520. In various embodiments, the steam 518 may be utilized to operate other components of the C/C preform production process 502. During the operations performed by the burner/steam generator 514, in addition to generating the steam 518, the burner/steam generator 514 generates byproducts 522, such as such as CO₂, H₂O, and N₂, among others, which the burner/steam generator 514 feeds into separator 524. Separator 524 may operate to separate the CO₂ 526 from the byproducts 522, which is fed into methanization reactor 510, to separate the N₂ 528 from the byproducts 522, which may be released into the atmosphere, and to separate the H₂O 530 from the byproducts 522, which may be fed into the burner/steam generator 514 along with H₂O 420. The separator 524 may include one or more of absorption, adsorption, cryogenic distillation, captured by solid sorbents or solvents such as mono-ethanol amine (MEA), or membrane separation, among others for the separation process.

In various embodiments, the methanization reactor 510 utilizes the incoming H₂ 508 provided via green H₂ supply 540 and CO₂ 526 to perform a methanization process in order to produce methane (CH₄) 532, which is fed into the C/C preform production process 502 or another system to perform various operation in the production the aircraft brakes, such as heat generation. In various embodiments, green H₂ supply 540 may be H₂ generated sustainably via electrolysis from a green H₂ supply device, such as a solar generator or wind generator. In various embodiments, in the production of CH₄ 532, the methanization reactor 510 also produces steam 534, which is fed into condenser 536. In various embodiments, the condenser 536 condenses the steam 534 to generate H₂O 538, which may be fed into the burner/steam generator 514 along with H₂O 520 and H₂O 530. Accordingly, system 500 provides for CO₂ emission recovery by converting the recovered CO₂ into other products that may be utilized by the C/C preform production process 502.

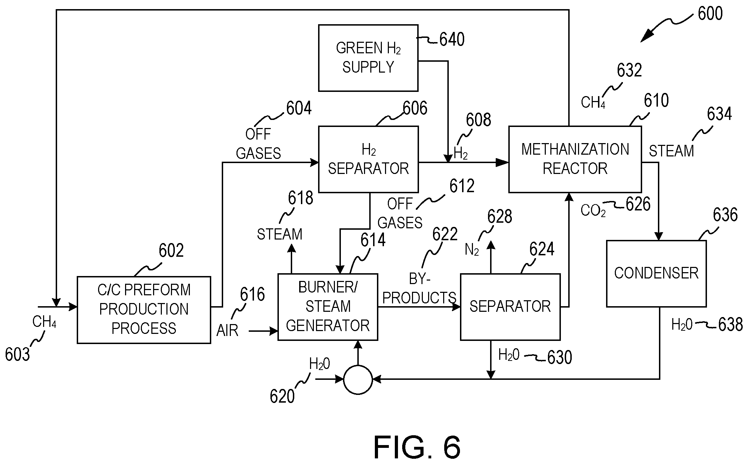

Referring now to FIG. 6, in accordance with various embodiments, a system 600 for CO₂ emission recovery by converting recovered CO₂ into other products is illustrated. In various embodiments, the C/C preform production process 602 utilizes natural gases such as methane (CH₄) 603 to perform various operations in the production of aircraft brakes. However, in various embodiments, during the C/C preform production process 602, various off gases 604, i.e. hydrocarbons, are produced. Accordingly, in system 600, the C/C preform production process 602 feeds the various off gases 604, i.e., CO₂, H₂, and nitrogen (N₂), among others, into H₂ separator 606. The H₂ separator 606 separates H₂ 608 from the various off gases 604 using one or more of membrane separation, pressure swing absorption (PSA), captured by solid sorbents or solvents such as mono-ethanol amine (MEA), or cryogenic distillation, among others. In various embodiments, the H₂ separator 606 feeds the H₂ 608 into methanization reactor 610 and feeds the remaining off gases 612, i.e. heavier hydrocarbons, i.e., CO₂ and N₂, among others, into burner/steam generator 614.

In various embodiments, the burner/steam generator 614 burns the remaining off gases 612 utilizing air 616 to generate steam 618 from incoming water (H₂O) 620. In various embodiments, the steam 618 may be utilized to operate other components of the C/C preform production process 602. During the operations performed by the burner/steam generator 614, in addition to generating the steam 618, the burner/steam generator 614 generates byproducts 622, such as such as CO₂, H₂O, and N₂, among others, which the burner/steam generator 614 feeds into separator 624. Separator 624 may operate to separate the CO₂ 626 from the byproducts 622, which is fed into methanization reactor 610, to separate the N₂ 628 from the byproducts 622, which may be released into the atmosphere, and to separate the H₂O 630 from the byproducts 622, which may be fed into the burner/steam generator 614 along with H₂O 620. The separator 624 may include one or more of absorption, adsorption, cryogenic distillation, captured by solid sorbents or solvents such as mono-ethanol amine (MEA), or membrane separation, among others for the separation process.

In various embodiments, the methanization reactor 610 utilizes the incoming H₂ 408, which may be supplemented by green H₂ supply 640, and CO₂ 626 to perform a methanization process in order to produce methane (CH₄) 632, which is fed into the C/C preform production process 602 or another system to perform various operation in the production the aircraft brakes, such as heat generation. In various embodiments, green H₂ supply 640 may be H₂ generated sustainably via electrolysis from a green H₂ supply device, such as a solar generator or wind generator. In various embodiments, in the production of CH₄ 632, the methanization reactor 610 also produces steam 634, which is fed into condenser 636. In various embodiments, the condenser 636 condenses the steam 634 to generate H₂O 638, which may be fed into the burner/steam generator 614 along with H₂O 620 and H₂O 630. Accordingly, system 600 provides for CO₂ emission recovery by converting the recovered CO₂ into other products that may be utilized by the C/C preform production process 602.

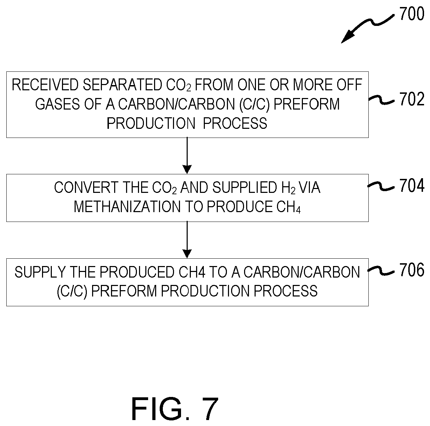

Referring now to FIG. 7, in accordance with various embodiments, a method for CO₂ emission recovery by converting recovered CO₂ into other products is illustrated. For ease of description, the method 700 is described with reference to FIGS. 4 thru 6. At block 702, a methanization reactor receives CO₂ separated from one or more off gases of a carbon/carbon (C/C) preform production process. At block 704, the methanization reactor converts the CO₂ and supplied H₂ via methanization to produce CH₄. At block 706, the methanization reactor supplies the produced CH₄ to a carbon/carbon (C/C) preform production process.

Benefits and other advantages have been described herein with regard to specific embodiments. Furthermore, the connecting lines shown in the various figures contained herein are intended to represent exemplary functional relationships and/or physical couplings between the various elements. It should be noted that many alternative or additional functional relationships or physical connections may be present in a practical system. However, the benefits, advantages, and any elements that may cause any benefit or advantage to occur or become more pronounced are not to be construed as critical, required, or essential features or elements of the disclosure. The scope of the disclosure is accordingly to be limited by nothing other than the appended claims, in which reference to an element in the singular is not intended to mean "one and only one" unless explicitly so stated, but rather "one or more." Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C.

Systems, methods, and apparatus are provided herein. In the detailed description herein, references to "one embodiment," "an embodiment," "various embodiments," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

Numbers, percentages, or other values stated herein are intended to include that value, and also other values that are about or approximately equal to the stated value, as would be appreciated by one of ordinary skill in the art encompassed by various embodiments of the present disclosure. A stated value should therefore be interpreted broadly enough to encompass values that are at least close enough to the stated value to perform a desired function or achieve a desired result. The stated values include at least the variation to be expected in a suitable industrial process, and may include values that are within 10%, within 5%, within 1%, within 0.1%, or within 0.01% of a stated value. Additionally, the terms "substantially," "about," or "approximately" as used herein represent an amount close to the stated amount that still performs a desired function or achieves a desired result. For example, the term "substantially," "about," or "approximately" may refer to an amount that is within 10% of, within 5% of, within 1% of, within 0.1% of, and within 0.01% of a stated amount or value.

Furthermore, no element, component, or method step in the present disclosure is intended to be dedicated to the public regardless of whether the element, component, or method step is explicitly recited in the claims. As used herein, the terms "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus.

Finally, it should be understood that any of the above-described concepts can be used alone or in combination with any or all of the other above-described concepts. Although various embodiments have been disclosed and described, one of ordinary skill in this art would recognize that certain modifications would come within the scope of this disclosure. Accordingly, the description is not intended to be exhaustive or to limit the principles described or illustrated herein to any precise form. Many modifications and variations are possible in light of the above teaching.

## Claims

1. A system for carbon dioxide (CO₂) emission recovery, the system comprising:
a methanization reactor (410, 510, 610), wherein the methanization reactor (410, 510, 610) is configured to:
receive CO₂ separated from one or more off gases of a carbon/carbon (C/C) preform production process;
convert the CO₂ and supplied hydrogen (H₂) via methanization to produce methane (CH₄); and
supply the produced CH₄ to at least one of the carbon/carbon (C/C) preform production process or another system for heat generation, and
further comprising:
an H₂ separator (406, 606), wherein the supplied H₂ is separated by the H₂ separator from the one or more off gases produced from the C/C preform production process.

2. The system of claim 1, further comprising:
a green H₂ supply device, wherein the supplied H₂ is green H₂ supplied via the green H₂ supply device and wherein the green H₂ supply device is at least one of a solar generator or wind generator, or further comprising:
a green H₂ supply device, wherein a portion of the supplied H₂ is separated by the H₂ separator from the one or more off gases produced from the C/C preform production process and is supplemented by green H₂ supplied via the green H₂ supply device and wherein the green H₂ supply device is a solar generator or wind generator.

3. The system of claim 1, further comprising:
a burner/steam generator (414,514,614), wherein remaining off gases from the separation of the supplied H₂ are fed to the burner/steam generator (414,514,614) and wherein the burner/steam generator (414,514,614) is configured to generate at least the CO₂ that is used by the methanization reactor (410, 510, 610).

4. The system of claim 3, wherein the burner/steam generator (414,514,614) further is configured to generate steam and wherein the steam is supplied to the C/C preform production process.

5. The system of claim 3, further comprising:
a separator, wherein the burner/steam generator (414,514,614) is configured to generate byproducts and wherein the separator is configured to separate the CO₂ from the byproducts and to provide the CO₂ to the methanization reactor (410, 510, 610).

6. The system of claim 5, wherein the separator further is configured to separate nitrogen (N₂) from the byproducts and to release the N₂ into an atmosphere.

7. The system of claim 5, wherein the separator further is configured to separate water (H₂O) from the byproducts and to feed the H₂O into the burner/steam generator (414,514,614).

8. The system of any preceding claim, further comprising:
a burner/steam generator (414,514,614); and
a condenser (436,536,636), wherein converting the CO₂ and the supplied hydrogen (H₂) via methanization produces the CH₄ and steam and wherein the condenser (436,536,636) is configured to condense the steam to water (H₂O) which is supplied to the burner/steam generator (414,514,614).

9. A method for carbon dioxide (CO₂) emission recovery, the method comprising:
receiving (702), by a methanization reactor (410, 510, 610), CO₂ separated from one or more off gases of a carbon/carbon (C/C) preform production process;
converting (704), by the methanization reactor (410, 510, 610), the CO₂ and supplied hydrogen (H₂) via methanization to produce methane (CH₄); and
supplying (706), by the methanization reactor (410, 510, 610), the produced CH₄ to at least one the carbon/carbon (C/C) preform production process or another system for heat generation.

10. The method of claim 9, wherein the supplied H₂ is separated, by an H₂ separator, from the one or more off gases produced from the C/C preform production process prior to the methanization reactor (410, 510, 610) converting the CO₂ and the supplied H₂ via methanization to produce the CH₄, or wherein the supplied H₂ is green H₂ supplied via a green H₂ supply device and wherein the green H₂ supply device is at least one of a solar generator or wind generator, or wherein a portion of the supplied H₂ is provided by separating, by an H₂ separator, the supplied H₂ from the one or more off gases produced from the C/C preform production process prior to the methanization reactor (410, 510, 610) converting the CO₂ and the supplied H₂ via methanization to produce the CH₄ and wherein the portion of the supplied H₂ is supplemented by green H₂ supplied via a green H₂ supply device and wherein the green H₂ supply device is a solar generator or wind generator.

11. The method of claim 9, wherein the supplied H₂ is separated, by a H₂ separator, from the one or more off gases produced from the C/C preform production process and remaining off gases from the separation of the supplied H₂ are fed to a burner/steam generator (414,514,614) and wherein at least the CO₂ is generated by the burner/steam generator (414,514,614) and used by the methanization reactor (410, 510, 610).

12. The method of claim 11, wherein steam is generated by the burner/steam generator (414,514,614) and wherein the steam is supplied to the C/C preform production process.

13. The method of claim 11, wherein the burner/steam generator (414,514,614) generates byproducts and wherein a separator separates the CO₂ from the byproducts and provides the CO₂ to the methanization reactor (410, 510, 610).

14. The method of claim 13, wherein the separator further separates nitrogen (N₂) from the byproducts and releases the N₂ into an atmosphere, or wherein the separator further separates water (H₂O) from the byproducts and feeds the H₂O into the burner/steam generator (414,514,614).

15. The method of claim 9, wherein converting the CO₂ and the supplied hydrogen (H₂) via methanization produces the CH₄ and steam and wherein a condenser (436,536,636) condenses the steam to water (H₂O) which is supplied to a burner/steam generator (414,514,614).

## Patentansprüche

1. System zur Rückgewinnung von Kohlendioxid(CO₂) -Emissionen, wobei das System Folgendes umfasst:
einen Methanisierungsreaktor (410, 510, 610), wobei der Methanisierungsreaktor (410, 510, 610) zu Folgendem konfiguriert ist:
Empfangen von CO₂, das aus einem oder mehreren Abgasen eines Kohlenstoff/Kohlenstoff(C/C)-Vorformherstellungsvorgangs abgeschieden wird;
Umwandeln des CO₂ und des zugeführten Wasserstoffs (H₂) über Methanisierung, um Methan (CH₄) herzustellen; und
Zuführen des hergestellten CH₄ zu mindestens einem von dem Kohlenstoff/Kohlenstoff(C/C)-Vorformherstellungsvorgang oder einem anderen System zur Wärmegenerierung, und
ferner umfassend:
einen H₂-Abscheider (406, 606), wobei das zugeführte H₂ durch den H₂-Abscheider aus dem einen oder den mehreren Abgasen, die aus dem C/C-Vorformherstellungsvorgang hergestellt werden, abgeschieden wird.

2. System nach Anspruch 1, ferner umfassend:
eine grüne H₂-Zuführvorrichtung, wobei das zugeführte H₂ grünes H₂ ist, das über die grüne H₂-Zuführvorrichtung zugeführt wird, und wobei die grüne H₂-Zuführvorrichtung mindestens eines von einem Solargenerator oder einem Windgenerator ist, oder ferner umfassend:
eine grüne H₂-Zuführvorrichtung, wobei ein Teil des zugeführten H₂ durch den H₂-Abscheider aus dem einen oder den mehreren Abgasen, die aus dem C/C-Vorformherstellungsvorgang hergestellt werden, abgeschieden wird und durch grünes H₂ ergänzt wird, das über die grüne H₂-Zuführvorrichtung zugeführt wird, und wobei die grüne H₂-Zuführvorrichtung ein Solargenerator oder ein Windgenerator ist.

3. System nach Anspruch 1, das ferner Folgendes umfasst:
einen Brenner/Dampfgenerator (414, 514, 614), wobei die verbleibenden Abgase aus der Abscheidung des zugeführten H₂ in den Brenner/Dampfgenerator (414, 514, 614) eingespeist werden und wobei der Brenner/Dampfgenerator (414, 514, 614) konfiguriert ist, mindestens das CO₂ zu generieren, das durch den Methanisierungsreaktor (410, 510, 610) verwendet wird.

4. System nach Anspruch 3, wobei der Brenner/Dampfgenerator (414, 514, 614) ferner konfiguriert ist, Dampf zu generieren, und wobei der Dampf dem C/C-Vorformherstellungsvorgang zugeführt wird.

5. System nach Anspruch 3, ferner umfassend:
einen Abscheider, wobei der Brenner/Dampfgenerator (414, 514, 614) konfiguriert ist, Nebenprodukten zu generieren und wobei der Abscheider konfiguriert ist, das CO₂ von den Nebenprodukten abzuscheiden und das CO₂ dem Methanisierungsreaktor (410, 510, 610) bereitzustellen.

6. System nach Anspruch 5, wobei der Abscheider ferner konfiguriert ist, Stickstoff (N₂) von den Nebenprodukten abzuscheiden und das N₂ in eine Atmosphäre freizusetzen.

7. System nach Anspruch 5, wobei der Abscheider ferner konfiguriert ist, Wasser (H₂O) von den Nebenprodukten abzuscheiden und das H₂O in den Brenner/Dampfgenerator (414, 514, 614) einzuspeisen.

8. System nach einem der vorhergehenden Ansprüche, ferner umfassend:
einen Brenner/Dampfgenerator (414, 514, 614); und
einen Kondensator (436, 536, 636), wobei durch Umwandeln des CO₂ und des zugeführten Wasserstoffs (H₂) über Methanisierung das CH₄ und Dampf hergestellt werden und wobei der Kondensator (436, 536, 636) konfiguriert ist, den Dampf zu Wasser (H₂O) zu kondensieren, das dem Brenner/Dampfgenerator (414, 514, 614) zugeführt wird.

9. Verfahren zur Rückgewinnung von Kohlendioxid(CO₂)-Emissionen, wobei das Verfahren Folgendes umfasst:
Empfangen (702) von CO₂, das aus einem oder mehreren Abgasen eines Kohlenstoff/Kohlenstoff(C/C)-Vorformherstellungsvorgangs abgeschieden wird, durch einen Methanisierungsreaktor (410, 510, 610);
Umwandeln (704) des CO₂ und des zugeführten Wasserstoffs (H₂) über Methanisierung durch den Methanisierungsreaktor (410, 510, 610), um Methan (CH₄) herzustellen; und
Zuführen (706) des hergestellten CH₄ durch den Methanisierungsreaktor (410, 510, 610) zu mindestens einem der Kohlenstoff/Kohlenstoff(C/C)-Vorformherstellungsvorgänge oder einem anderen System zur Wärmegenerierung.

10. Verfahren nach Anspruch 9, wobei das zugeführte H₂ durch einen H₂-Abscheider aus dem einen oder den mehreren Abgasen, die aus dem C/C-Vorformherstellungsvorgang hergestellt werden, abgeschieden wird, bevor der Methanisierungsreaktor (410, 510, 610) das CO₂ und das zugeführte H₂ über Methanisierung umwandelt, um CH₄ herzustellen, oder wobei das zugeführte H₂ grünes H₂ ist, das über eine grüne H₂-Zuführvorrichtung zugeführt wird, und wobei die grüne H₂-Zuführvorrichtung mindestens eines von einem Solargenerator oder einem Windgenerator ist, oder wobei ein Teil des zugeführten H₂ bereitgestellt wird, indem das zugeführte H₂ durch einen H₂-Abscheider aus dem einen oder den mehreren Abgasen, die aus dem C/C-Vorformherstellungsvorgang hergestellt werden, abgeschieden wird, bevor der Methanisierungsreaktor (410, 510, 610) das CO₂ und das zugeführte H₂ über Methanisierung umwandelt, um CH₄ herzustellen, und wobei der Teil des zugeführten H₂ durch grünes H₂ ergänzt wird, das über eine grüne H₂-Zuführvorrichtung zugeführt wird, und wobei die grüne H₂-Zuführvorrichtung ein Solargenerator oder ein Windgenerator ist.

11. Verfahren nach Anspruch 9, wobei das zugeführte H₂ durch einen H₂-Abscheider aus dem einen oder den mehreren Abgasen, die aus dem C/C-Vorformherstellungsvorgang hergestellt werden, abgeschieden wird und die verbleibenden Abgase aus der Abscheidung des zugeführten H₂ in einen Brenner/Dampfgenerator (414, 514, 614) eingespeist werden und wobei mindestens das CO₂ durch den Brenner/Dampfgenerator (414, 514, 614) generiert und durch den Methanisierungsreaktor (410, 510, 610) verwendet wird.

12. Verfahren nach Anspruch 11, wobei Dampf durch den Brenner/Dampfgenerator (414, 514, 614) generiert wird und wobei der Dampf dem C/C-Vorformherstellungsvorgang zugeführt wird.

13. Verfahren nach Anspruch 11, wobei der Brenner/Dampfgenerator (414, 514, 614) Nebenprodukte generiert und wobei ein Abscheider das CO₂ von den Nebenprodukten abscheidet und das CO₂ dem Methanisierungsreaktor (410, 510, 610) bereitstellt.

14. Verfahren nach Anspruch 13, wobei der Abscheider ferner Stickstoff (N₂) von den Nebenprodukten abscheidet und das N₂ in eine Atmosphäre freisetzt, oder wobei der Abscheider ferner Wasser (H₂O) von den Nebenprodukten abscheidet und das H₂O in den Brenner/Dampfgenerator (414, 514, 614) einspeist.

15. Verfahren nach Anspruch 9, wobei durch Umwandeln des CO₂ und des zugeführten Wasserstoffs (H₂) über Methanisierung das CH₄ und Dampf hergestellt werden und wobei ein Kondensator (436, 536, 636) den Dampf zu Wasser (H₂O) kondensiert, das einem Brenner/Dampfgenerator (414, 514, 614) zugeführt wird.

## Revendications

1. Système de récupération des émissions de dioxyde de carbone (CO₂), le système comprenant :
un réacteur (410, 510, 610) de méthanisation, dans lequel le réacteur (410, 510, 610) de méthanisation est configuré pour :
la réception de CO₂ séparé d'un ou plusieurs gaz de dégagement d'un procédé de production de préforme de carbone-carbone (C/C) ;
la conversion par méthanisation du CO₂ et de l'hydrogène fourni (H₂) pour produire du méthane (CH₄) ; et
la fourniture du CH₄ produit à au moins l'un du procédé de production de préforme de carbone-carbone (C/C) ou d'un autre système de production de chaleur, et
comprenant en outre :
un séparateur (406, 606) de H₂, dans lequel le H₂ fourni est séparé par le séparateur de H₂ des un ou plusieurs gaz de dégagement produits par le procédé de production de préforme de C/C.

2. Système selon la revendication 1, comprenant en outre :
un dispositif d'alimentation en H₂ vert, dans lequel le H₂ fourni est du H₂ vert produit par le dispositif d'alimentation en H₂ vert et dans lequel le dispositif d'alimentation en H₂ vert est au moins l'un d'un générateur solaire ou d'un générateur éolien, ou comprenant en outre :
un dispositif d'alimentation en H₂ vert, dans lequel une partie du H₂ fourni est séparée par le séparateur de H₂ des un ou plusieurs gaz de dégagement produits par le procédé de production de préforme de C/C et est complétée par du H₂ vert fourni par le dispositif d'alimentation en H₂ vert et dans lequel le dispositif d'alimentation en H₂ vert est un générateur solaire ou un générateur éolien.

3. Système selon la revendication 1, comprenant en outre :
un brûleur/générateur (414, 514, 614) de vapeur, dans lequel les gaz de dégagement restants de la séparation du H₂ fourni sont acheminés vers le brûleur/générateur (414, 514, 614) de vapeur et dans lequel le brûleur/générateur (414, 514, 614) de vapeur est configuré pour générer au moins le CO₂ utilisé par le réacteur (410, 510, 610) de méthanisation.

4. Système selon la revendication 3, dans lequel le brûleur/générateur (414, 514, 614) de vapeur est en outre configuré pour générer de la vapeur et dans lequel la vapeur est fournie au procédé de production de préforme de C/C.

5. Système selon la revendication 3, comprenant en outre :
un séparateur, dans lequel le brûleur/générateur (414, 514, 614) de vapeur est configuré pour générer des sous-produits et dans lequel le séparateur est configuré pour séparer le CO₂ des sous-produits et pour fournir le CO₂ au réacteur (410, 510, 610) de méthanisation.

6. Système selon la revendication 5, dans lequel le séparateur est en outre configuré pour séparer l'azote (N₂) des sous-produits et pour libérer le N₂ dans l'atmosphère.

7. Système selon la revendication 5, dans lequel le séparateur est en outre configuré pour séparer l'eau (H₂O) des sous-produits et pour acheminer le H₂O vers le brûleur/générateur (414, 514, 614).

8. Système selon une quelconque revendication précédente, comprenant en outre :
un brûleur/générateur (414, 514, 614) de vapeur ; et
un condenseur (436, 536, 636), dans lequel la conversion par méthanisation du CO₂ et de l'hydrogène (H₂) fourni produit du CH₄ et de la vapeur et dans lequel le condenseur (436, 536, 636) est configuré pour condenser la vapeur en eau (H₂O) qui est fournie au brûleur/générateur (414, 514, 614) de vapeur.

9. Procédé de récupération des émissions de dioxyde de carbone (CO₂), le procédé comprenant :
la réception (702), par un réacteur (410, 510, 610) de méthanisation, du CO₂ séparé d'un ou plusieurs gaz de dégagement d'un procédé de production de préforme de carbone-carbone (C/C) ;
la conversion (704) par méthanisation, par le réacteur (410, 510, 610) de méthanisation, du CO₂ et de l'hydrogène fourni (H₂) pour produire du méthane (CH₄) ; et
la fourniture (706), par le réacteur (410, 510, 610) de méthanisation, du CH₄ produit à au moins l'un du procédé de production de préforme de carbone-carbone (C/C) ou d'un autre système de production de chaleur.

10. Procédé selon la revendication 9, dans lequel le H₂ fourni est séparé, par un séparateur de H₂, des un ou plusieurs gaz de dégagement produits par le procédé de production de préforme de C/C avant la conversion par méthanisation du CO₂ et du H₂ fourni par le réacteur (410, 510, 610) de méthanisation pour produire du CH₄, ou dans lequel le H₂ fourni est du H₂ vert fourni par un dispositif d'alimentation en H₂ vert et dans lequel le dispositif d'alimentation en H₂ vert est au moins l'un d'un générateur solaire ou d'un générateur éolien, ou dans lequel une partie du H₂ fourni est fourni par la séparation, par un séparateur de H₂, du H₂ fourni des un ou plusieurs gaz de dégagement produits par le procédé de production de préforme de C/C avant la conversion par méthanisation du CO₂ et du H₂ fourni par le réacteur (410, 510, 610) de méthanisation pour produire le CH₄ et dans lequel la partie du H₂ fourni est complétée par le H₂ vert fourni par un dispositif d'alimentation en H₂ vert et dans lequel le dispositif d'alimentation en H₂ vert est un générateur solaire ou un générateur éolien.

11. Procédé selon la revendication 9, dans lequel le H₂ fourni est séparé, par un séparateur de H₂, des un ou plusieurs gaz de dégagement produits par le procédé de production de préforme de C/C et des gaz de dégagement restants de la séparation du H₂ fourni sont acheminés vers un brûleur/générateur (414, 514, 614) de vapeur et dans lequel au moins le CO₂ est généré par le brûleur/générateur (414, 514, 614) de vapeur et utilisé par le réacteur (410, 510, 610) de méthanisation.

12. Procédé selon la revendication 11, dans lequel la vapeur est générée par le brûleur/générateur (414, 514, 614) de vapeur et dans lequel la vapeur est fournie au procédé de production de préforme de C/C.

13. Procédé selon la revendication 11, dans lequel le brûleur/générateur (414, 514, 614) de vapeur génère des sous-produits et dans lequel un séparateur sépare le CO₂ des sous-produits et fournit le CO₂ au réacteur (410, 510, 610) de méthanisation.

14. Procédé selon la revendication 13, dans lequel le séparateur sépare en outre l'azote (N₂) des sous-produits et libère le N₂ dans l'atmosphère, ou dans lequel le séparateur sépare en outre l'eau (H₂O) des sous-produits et achemine le H₂O vers le brûleur/générateur (414, 514, 614) de vapeur.

15. Procédé selon la revendication 9, dans lequel la conversion par méthanisation du CO₂ et de l'hydrogène (H₂) fourni produit du CH₄ et de la vapeur et dans lequel un condenseur (436, 536, 636) condense la vapeur en eau (H₂O) qui est fournie à un brûleur/générateur (414, 514, 614) de vapeur.
